# EUROPÄISCHE PATENTSCHRIFT

(11) **EP 0 659 060 B1**
(45) Veröffentlichungstag und Bekanntmachung des Hinweises auf die Patenterteilung: **15.01.1997**
(21) Anmeldenummer: 93915967.9
(22) Anmeldetag: 21.07.1993
(51) Int. Cl.: A61B 17/12, A61B 17/122, A61B 17/128

(54) **CLIP ZUR ANWENDUNG IN DER CHIRURGIE UND CLIPAPPLIKATOR**
CLIP FOR SURGICAL USE AND CLIP APPLICATOR
PINCE POUR UTILISATION CHIRURGICALE ET SON APPLICATEUR

(30) Priorität: 09.09.1992 DE 4230102
(43) Veröffentlichungstag der Anmeldung: 28.06.1995
(73) Patentinhaber: Forschungszentrum Karlsruhe GmbH, 76133 Karlsruhe (DE)
(72) Erfinder: KUNTZ, Bertram, D-76889 Steinfeld (DE); SCHLIPF, Karl, D-76137 Karlsruhe (DE); SCHÜLKEN, Heinrich, D-76297 Stutensee-Staffort (DE)
(74) Vertreter: Rückert, Friedrich, Dr.
(86) Internationale Anmeldenummer: EP9301929
(87) Internationale Veröffentlichungsnummer: WO9405219

(56) Entgegenhaltungen:
- EP-A- 0 073 655
- EP-A- 0 201 344
- WO-A-80/01752
- DE-A- 2 402 677
- GB-A- 972 731
- GB-A- 2 025 511
- US-A- 4 556 060

## Beschreibung

Die Erfindung betrifft einen Clip zur Anwendung in der Chirurgie gemäß dem Oberbegriff des ersten Patentanspruchs und einen Clipapplikator zum Setzen des Clip.

In der Medizin sind eine Vielzahl von verschiedenartig geformten Clips und Applikatoren zum Setzen der Clips bekannt.

Ein Clip der eingangs genannten Art ist aus der US 4,498,476 bekannt. Dieser Clip, der zum Abklemmen von Blutgefäßen dient, besteht aus einem Clipoberteil und einem Clipunterteil, die durch ein biegeweiches Brückenteil symmetrisch miteinander verbunden sind. Das Clipoberteil enthält an der dem Brückenteil gegenüberliegenden Seite eine Öffnung, in die ein auf dem Clipunterteil angebrachter Stift einrastet. Der Stift trägt in einigen Ausführungsformen eine dachartig überkragende oder pfeilförmige Spitze. Der Stift und gegebenenfalls die Spitze wirken zusammen mit der Öffnung als Verriegelungselemente zum Schließen des Clip. Im geschlossenen Zustand berühren sich Clipoberteil und Clipunterteil. Mit diesem Clip lassen sich keine Geweberänder approximieren und fixieren.

Aus der DE 30 34 356 A1 ist eine chirurgische Klemme, insbesondere eine Blutgefäßklemme bekannt, die ebenfalls aus einem Oberteil und einem Unterteil besteht, wobei Ober- und Unterteil durch ein biegeweiches Brückenteil symmetrisch miteinander verbunden sind. Das Oberteil enthält an der dem Brückenteil gegenüberliegenden Seite eine Öffnung, in die eine Ratsche eingreifen kann. Die Ratsche trägt auf einer Seite Zähne, mit deren Hilfe die Klemme geschlossen und verriegelt werden kann. Auch mit dieser Klemme lassen sich keine Geweberänder approximieren und fixieren.

Im deutschen Gebrauchsmuster G 83 29 725 wird ein Mikroclip für medizinische Zwecke aus elastisch verformbarem Material beschrieben, der aus einem U-förmigen Teil mit an den freien Schenkeln des U angesetzten und etwa parallel zu den Schenkeln des U zurückverlaufenden, im wesentlichen geradlinigen Abschnitten besteht und dadurch gekennzeichnet ist, daß die geradlinigen Abschnitte im geringen Abstand von den Schenkeln des U verlaufen und diese nur in der Nähe der Verbindung mit den freien Schenkeln des U berühren. Der Mikroclip besteht vorzugsweise aus weich-elastischem rostfreien Stahl. Die freien Enden des U können Durchbrüche enthalten, an denen ein Clipapplikator angesetzt werden kann.

Der bekannte Mikroclip ist ebenfalls hauptsächlich zum Abklemmen von Blutgefäßen vorgesehen; zum Fixieren von Geweberändern nach Inzisionen erscheint er weniger geeignet.

Ein weiterer chirurgischer Mikroclip ist in der EP 0 432 743 A1 beschrieben. Dieser Mikroclip weist zwei einander gegenüberliegende Flansche aus Metallblech auf. An den Flanschen sind jeweils an einem Ende zueinander gerichtete Spitzen und am anderen Ende gegen Federkraft zusammendrückbare Griffbereiche angebracht. Der Mikroclip ist einstückig ausgebildet und besitzt einen sich zwischen den Griffbereichen erstreckenden Verbindungsflansch, der federnd elastisch ausgebildet ist.

Der Mikroclip muß, da er offensichtlich von Hand gesetzt wird, verhältnismäßig groß sein und eignet sich daher nicht für die internistische Chirurgie und insbesondere nicht für die minimal invasive Chirurgie (MIC).

Aus der DE 31 39 488 C2 ist ein Aneurysma-Clip bekannt, der zwei schwenkbar miteinander verbundene, sich überkreuzende Schenkel aufweist, mit deren Hilfe Gewebe fixiert werden kann. Die Schenkel werden mit Hilfe einer Doppelwindung eines Federdrahts, der mit den beiden Schenkeln verbunden ist, zusammengepreßt.

Ein ähnlicher Clip ist in der DE 35 23 031 A1 beschrieben. Bei diesem Clip wird die Doppelwindung des Federdrahts ersetzt durch eine Nabe bestehend aus zwei Nabenabschnitten, die durch einen Stift drehbar miteinander verbunden sind.

Die beiden zuletzt beschriebenen Clips eignen sich insbesondere zum temporären Abklemmen von Blutgefäßen; zur dauerhaften Fixierung von Geweberändern nach Inzisionen sind sie nicht geeignet.

Aus der DE 30 28 259 A1 ist eine Tubenligaturpistole bekannt, mit der sich Clips zur Unterbrechung von Gefäßen setzen lassen. Die Vorrichtung eignet sich für Clips, deren Ober- und Unterteil über eine Achse gegeneinander klappbar sind und die sich durch eine Feder fixieren lassen.

Eine Clipanlegezange ist aus der DE 40 24 636 A1 bekannt. Diese Clipanlegezange weist ein Rohr auf, das mit Hilfe von zwei Griffhälften in axialer Richtung bewegt werden kann. Am distalen Ende des Rohres stehen zwei Klauen vor, die über ein Seil, das in dem Rohr geführt ist, geöffnet und geschlossen werden können. Das Rohr besteht aus einer schlauchförmigen elastischen Umhüllung, die beim Verfahren des Seils positionsstabil bleibt. Die Clipanlegezange dient offensichtlich zum Anlegen von Aneurysma-Clips.

Eine weitere Zange zum Anlegen von blutstillenden Clip wird in der DE 40 15 562 A1 beschrieben. Diese Zange weist an ihrem distalen Ende zwei Maulteile auf, die über eine Betätigungsstange in einem Schaft durch Betätigungsgriffe am proximalen Ende bewegbar sind. Die Betätigungsstange ist hohl und dient als Magazin zur Aufnahme einer größeren Anzahl von Clips. Mit dieser Zange können nur U-förmige Clip gesetzt werden.

Der Erfindung liegt die Aufgabe zugrunde, einen Clip der eingangs genannten Art und einen zum Setzen dieses Clip geeigneten Clipapplikator vorzuschlagen, mit deren Hilfe in der minimal invasiven Chirurgie (MIC) durch eine Trokarhülse Geweberänder nach Läsionen oder Inzisionen approximiert und fixiert werden können.

Die Aufgabe wird erfindungsgemäß durch die im Kennzeichen des ersten Patentanspruchs beschriebenen Merkmale und durch den im sechsten Patentanspruch beschriebenen zugehörigen Clipapplikator gelöst. In den abhängigen Ansprüchen sind vorteilhafte Ausführungsformen des erfindungsgemäßen Clip angegeben. Die Erfindung wird im folgenden anhand von Figuren erläutert. Es zeigen:
Fig. 1 einen erfindungsgemäßen Clip in Seitenansicht und in Draufsicht nach dem Setzen in (nicht dargestelltein) Gewebe;
Fig. 2 das distale Ende des Applikators mit einem gefaßten Clip in Draufsicht;
Fig. 3 das distale Ende des Applikators mit einem eingelegten Clip in der Ausgangsform in Seitenansicht;
Fig. 4 das distale Ende des Applikators mit einem verformten, durch eine Trokarhülse einschiebbaren Clip in Seitenansicht;
Fig. 5 das distale Ende des Applikators mit einem teilweise geöffneten, gebrauchsbereiten Clip in Seitenansicht;
Fig. 6 den Clipapplikator in Seitenansicht;
Fig. 7 das distale Ende des Applikators und den verformten Clip nach Approximierung und Fixierung von (nicht dargestellen) Geweberändern;
Fig. 8 das distale Ende des Applikators und den verformten und gegen Öffnen gesicherten Clip nach Approximierung und Fixierung von (nicht dargestelltem) Gewebe.

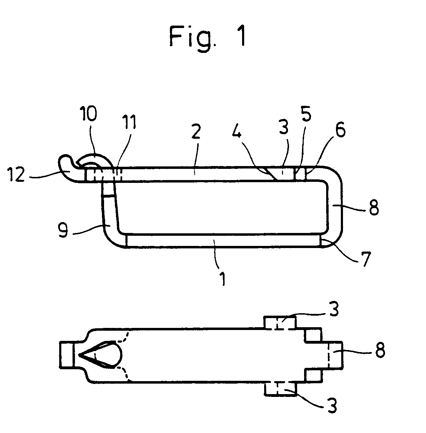

Der erfindungsgemäße Clip ist in seiner endgültigen, im Gewebe gesetzten Form in Fig. 1 dargestellt. Er besteht aus einem Clipunterteil 1 und einem Clipoberteil 2, die durch ein biegeweiches Brückenteil 8 miteinander verbunden sind.

Der Clip kann insgesamt aus einem verbiegbaren Material, etwa aus einem geeigneten Edelstahldraht mit einheitlichem Durchmesser bestehen. Beim Setzen des Clip wird in diesem Fall das Brückenteil durch einen entsprechend konstruierten Applikator verformt, während das Clipunterteil und das Clipoberteil unverformt bleiben. Vorzugsweise ist jedoch der Querschnitt des Brückenteils 8 gegenüber den Querschnitten der übrigen Teile des Clip vermindert. Hierdurch wird erreicht, daß der Stift 9 beim Setzen des Clip ohne zu verbiegen eine hohe Kraft auf das Gewebe ausüben kann, während der Kraftaufwand für das Verbiegen des Brückenteils in Grenzen bleibt.

Der erfindungsgemäße Clip hat eine Symmetrieebene. Das Clipunterteil 1 trägt an seinem freien Ende einen in der Symmetrieebene abgewinkelten Stift 9, der in eine Spitze 10 ausläuft. Das Clipoberteil 2 trägt an seinem freien Ende eine verrundete Protektornase 12. In der Nähe der Protektornase 12 ist das Clipoberteil 2 mit einem Durchbruch 11 versehen, der in der Symmetrieebene angeordnet ist. Die Längen des Clipunterteils 1 mit dem Stift 9, des biegeweichen Brückenteils 8 und des Clipoberteils 2 bis zum Durchbruch 11 sind so bemessen, daß der Stift 9 mit seiner Spitze 10 in den Durchbruch 11 eingreift, wenn das Clipoberteil 2 unter Verformung des Brückenteils 8 gegen das Clipunterteil 1 gedrückt wird.

Mit der Spitze 10 des Stiftes 9 wird das zu approximierende und zu fixierende Gewebe durchstochen und aufgeladen. Danach wird das Clipoberteil 2 durch Verbiegen des Brückenteils 8 so weit gegen den Stift 9 des Clipunterteils 1 gedrückt, bis die Spitze 10 durch den Durchbruch 11 greift. Der Querschnitt der Stiftspitze 10 und der Querschnitt des Durchbruchs wird einander entsprechend gewählt. Die einfachste Lösung besteht in einem kreisrunden Durchbruch 11 und einer kegel- oder pyramidenförmigen Spitze 10.

Vorzugsweise ist die Protektornase 12 in die selbe Richtung abgebogen wie der Stift 9. Hierdurch ist es möglich, den Clip gegen unbeabsichtigtes Öffnen zu sichern und gleichzeitig die Stiftspitze 10 so zu verwahren, daß weitere Verletzungen des fixierten Gewebes ausgeschlossen sind. Hierzu wird die aus dem Durchbruch 11 herausragende Stiftspitze 10 in Richtung auf die Protektornase 12 umgeknickt, so daß sie durch die abgebogene Protektornase 12 geschützt ist.

Die Konstruktion eines geeigneten Applikators wird erleichtert, wenn der Brückenteil 8 des Clip einen geringeren Querschnitt aufweist und Versatzflächen 6, 7 im Clipoberteil 2 und Clipunterteil 1 an der Grenzfläche zwischen diesen Clipteilen und dein Brückenteil 8 vorgesehen werden. Vorteilhaft ist weiterhin, wenn in dem dem Brückenteil 8 benachbarten Bereich des Clipoberteils 2 beidseitig ein Zapfen 3 senkrecht zur Symmetrieebene ausgeformt ist. Besonders bevorzugt sind Zapfen mit einer Abschrägung 4 auf der dem Brückenteil 8 abgewandten Seite.

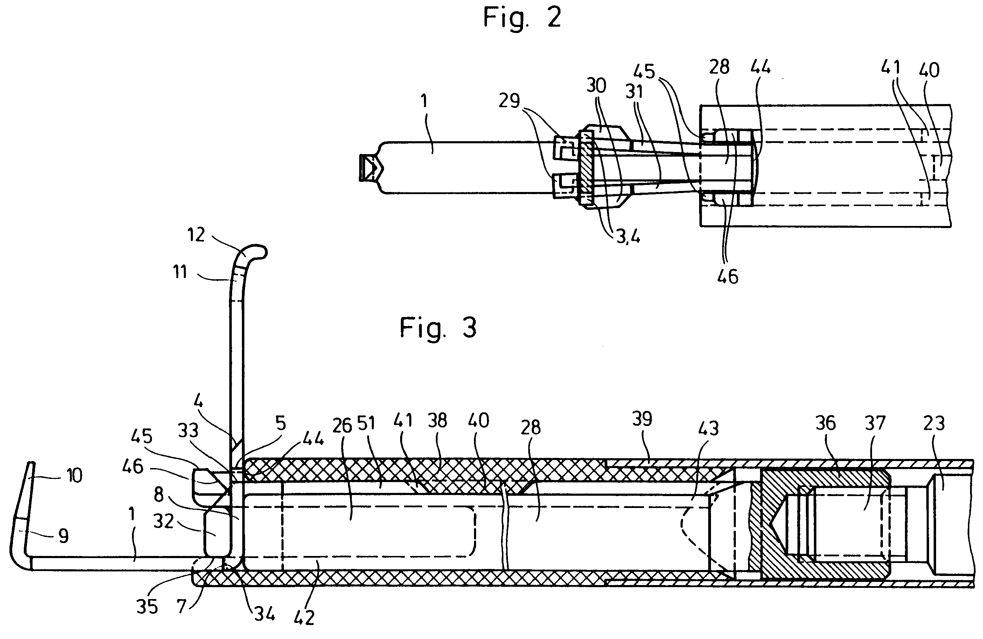

Der erfindungsgemäße Clip kann aus Blech gestanzt und anschließend in die in Fig. 3 dargestellte Ausgangsform gebogen werden. Er besteht aus einem einzigen Teil, so daß kein Zusammenfügen von kleinen Einzelteilen notwendig ist. Bei entsprechender Materialwahl, z. B. einem biegeweichen Edelstahl, kann der Brückenteil mehrmals verformt werden, ohne daß ein Bruch auftritt. Der Clip kann so klein gefertigt werden, daß er durch eine der gebräuchlichen Trokarhülsen mit 5, 7 oder 10 mm gesetzt werden kann.

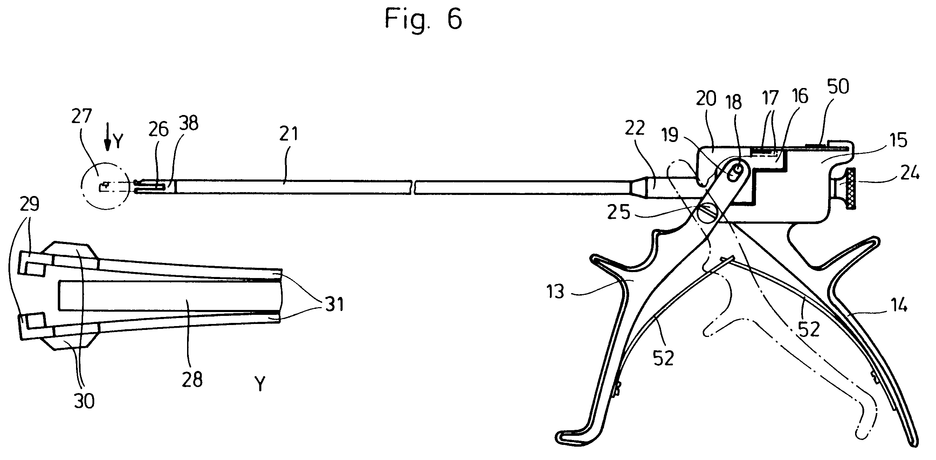

Die Konstruktion des an den erfindungsgemäßen Clip angepaßten Clipapplikators ist am besten aus den Fig. 3 und 6 ersichtlich. Wesentlich sind die folgenden Merkmale:
a) ein Clipgreifer zum Ergreifen und Festhalten des biegeweichen Brückenteils 8 des Clip;
b) ein Applikator-Schaftrohr 39, das über einen Betätigungsgriff 13, 14 axial und senkrecht zum festgehaltenen biegeweichen Brückenteil 8 des Clip verschiebbar ist und dessen Kopf 38 den Clip bei distalem Vorschub im Bereich des biegeweichen Brückenteils 8 in der Weise verformt, daß das Clipunterteil 1 und das Clipoberteil 2 eine angenähert parallele Stellung einnehmen;
c) zwei Öffnerzapfen 47 im Applikatorkopf 38 und eine Anschlagstange 28 im Clipgreifer 27, mit deren Hilfe bei proximaler Bewegung des Applikator-Schaftrohres 39 das Clipoberteil 2 durch Verformen des biegeweichen Brückenteils 8 in eine annähernd senkrechte Stellung zum Clipunterteil 1 gebracht werden kann.

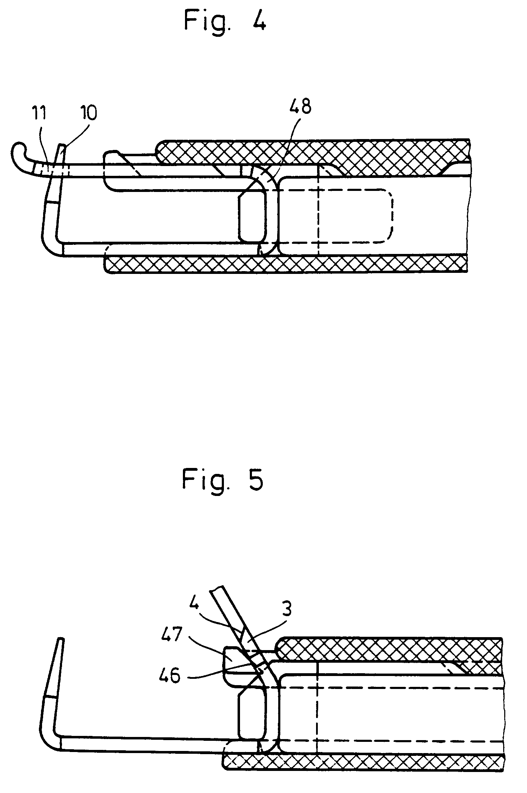

Die in den Fig. 2 bis 8 gezeigte Ausführungsform des Clipapplikators ist für Einhandbedienung konzipiert. Sie erlaubt das Einbringen des Clip in der in Fig. 4 dargestellten Form durch eine dem Applikatorkopf 38 und dem Applikatorschaft 21 angepaßte, Trokarhülse von 5, 7 oder 10 mm.

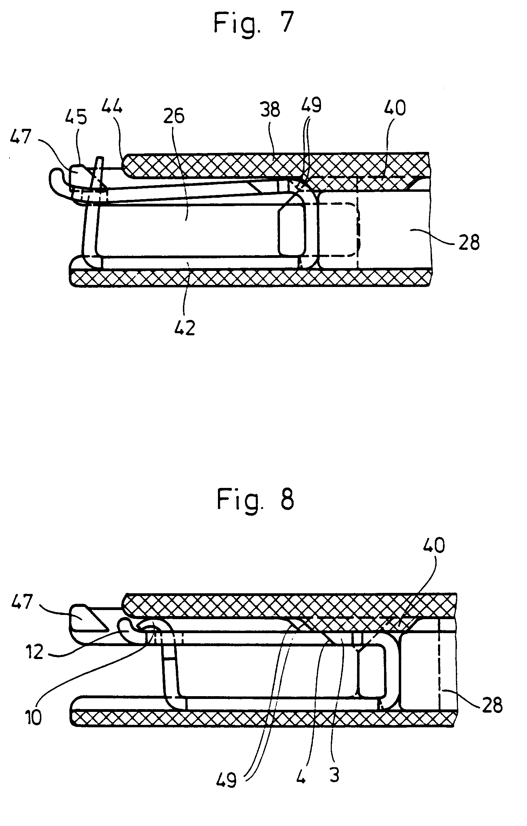

Durch einfache translatorische Bewegung des aus den Teilen 22, 38 und 39 bestehenden Schaftaußenteils relativ zum Schaftinnenteil 23 und zum Greifer 27 wird der Clip in der in Fig. 4 dargestellten Form gehalten und durch die Trokarhülse eingeführt, in die in Fig. 5 dargestellte Form geöffnet, nach Aufladen und Approximieren des Gewebes auf dem Stift 9 wie in Fig. 7 gezeigt geschlossen, wie in Fig. 8 dargestellt verriegelt und anschließend freigegeben. Das Schließen und Öffnen des Clip erfolgt unter Deformation des Scharnierbogens 48 (Fig. 4) im Brückenteil 8 durch das Kräftepaar zwischen der Greiferbacke 32 und der Anschlag- und Formungsrippe 44 beim Verschieben des Schaftrohres 39 in distaler Richtung (Schließen des Clip) bzw. durch das Kräftepaar zwischen der Anschlagstange 28 und den Öffnerzapfen 47 beim Zurückziehen des Schaftrohres 39 in proximale Richtung (Öffnen des Clip).

Clipapplikator und Clip sind so aufeinander abgestimmt, daß entsprechend Fig. 3 beim Ladevorgang die Zapfenrücken 5 an den Zapfen 3 des Clipoberteiles 2 mit genügend Spiel über die Deckflächen 45 der Öffnerzapfen 47 im Applikatorkopf 38 bis zur Anschlag- und Formungsrippe 44 hinweg bewegt werden können.

Beim Öffnen und Schließen bewegt sich der Clip translatorisch etwa zwischen der in Fig. 5 gezeigten Stellung und dem Beginn der Nachformungsrippen 40 und 41 im Applikatorkopf 38 (siehe Fig. 3). Durch die gezeigte Konstruktion des Applikatorkopfes 38 und des Greifers 27 ist erreicht, daß in diesem Arbeitsbereich der Radius des Scharnierbogens 48 relativ groß bleibt und seine plastischen Verformungen erst nach unrealistisch vielen Arbeitsspielen zum Materialversagen führen.

Beim Öffnen des Clip greift das oben beschriebene Kräftepaar am Brückenteil 8 und den beiden Zapfen 3 am Clipoberteil 2 an und führt so zu einer Radiusvergrößerung des Scharnierbogens 48.

Der Clip wird nach dem Greifen und Einziehen in den Applikatorkopf entsprechend Fig.3 durch Anlage an folgenden Einspann- oder Gleitflächen sicher gehalten und/oder geführt:
- Zwischen der distalen Stirnfläche der Anschlagstange 28 und den Greiferbacken 32,
- durch die Versatzflächen 7 am Clipunterteil 1 und die unteren Indexflächen 34 an den Greiferköpfen 29,
- durch die Versatzflächen 6 am Clipoberteil 2 und die Indexflächen 35 an den Greiferköpfen 29,
- durch die Führung des Clipunterteiles 1 sowohl seitlich in der Nut 42 des Applikatorkopfes 38 als auch senkrecht dazu durch die Führung zwischen den Unterseiten der Greiferbacken 32 und der Grundfläche der Nut 42,
- durch den seitlichen Einschluß des Brückenteils 8 in den Greiferbacken 29,
- durch die (vorübergehende) seitliche Führung des Clipoberteiles 2 zwischen den Öffnerzapfen 47 des Applikatorkopfes 38,
- durch die gleitende Anlage des Clipoberteiles 2 an der Grundfläche der Führungsnut 51 im Applikatorkopf 38 und
- durch die seitliche Führung der Clipzapfen 3 am Clipoberteil 2 in der Führungsnut 51 des Applikatorkopfes 38.

Das seitliche Ausweichen und Aufklaffen der Greiferbacken 29 in der Führungsnut 51 unter der Belastung der Clipumformungskräfte wird durch die Abstützung der Greiferbacken über die Führungsrippen 30 an den Seitenflächen der Führungsnut 51 verhindert.

Der Clipgreifer 27 und damit auch der gefaßte Clip sind über das Schaftinnenteil 23 und das Handtellergriffteil 14 durch die Hand des Operateurs ergonomisch günstig und sicher dirigierbar. Das Schließen und Öffnen des Clip im Operationssitus durch Verschieben des Applikatorkopfes 38 und des Schaftrohres 39 bei Betätigen des Fingergriffteiles 13 führt nicht zu einer Dislocierung des Clip, die stets durch eine korrigierende Handbewegung des Operateurs auszugleichen wäre.

Die Programmfolge beim Abarbeiten der oben aufgelisteten Systemfunktionen sowie die dazu erforderlichen Wege des Applikatorkopfes 38 relativ zum Clipgreifer 27 werden durch die Kulissenbahnen 17 im Antriebskopf 16 in Verbindung mit einer in sie eingreifenden Zunge am Federhebel 20 vorgegeben, der im Griffkopf 15 schwenkbar gelagert ist und der gegen den Druck der Flachfeder 50 zum Wechseln der Kulissenbahnen mit dem Daumen in Richtung Griffkopf 15 bewegt wird. Drei Arbeitsbereiche sind durch diese Programmechanik vorgegeben:

### Bereich 1:

### Greifen und Laden des Clip.

Der Applikatorkkopf 38 wird relativ zum Greifer 27 durch Betätigen des Finger-Griffteiles 13 aus der in Fig.6 gezeichneten Stellung in die Position nach Fig.5 gebracht. Das Fingergriffteil 13 ist dann gegen distales Rückstellen in die Ausgangslage durch die Flachfedern 52 blockiert.

### Bereich 2:

### Schließen und Öffnen des Clip.

Über das Fingergriffteil 13 kann der Applikatorkopf 38 relativ zum Greifer 27 zwischen zwei durch die Kulissenbahn festgelegten Anschlagpunkten hin und her bewegt werden. Dabei wird der Clip zum Aufladen von Geweberändern geöffnet (Fig.5) und geschlossen (Fig.4). Ein unbeabsichtigtes Freigeben oder Verriegeln des Clip ist nicht möglich.

### Bereich 3:

### Verriegeln und Freigeben des Clip.

Nach dem Aufladen beider Geweberänder auf den Stift 9 des Clip wird wie oben beschrieben per Daumen der Federhebel 20 betätigt. Dadurch springt die Zunge am Federhebel 20 in den tieferliegenden zweiten Teil der Kulissenbahn 17. Durch das weitere jetzt mögliche Anziehen des Fingergriffteiles 13 wird unter den Nachformungsrippen 40 und 41 der Scharnierbogen 48 umgeformt, das Clipoberteil 2 tiefer gelegt (Fig.7 und 8), die Stiftspitze 10 auf die Protektornase 12 umgebogen und dadurch der Clip verriegelt (Fig.8). Bei Entlastung des Griffteiles 13 wird durch die Wirkung der Flachfedern 52 der Applikatorkopf 38 nach proximal zurückgezogen und der Clip mit den fixierten Geweberändern freigegeben.

Nach Lösen und Entfernen der beiden Rändelschrauben 18 und 24 kann der Applikatorschaft 21 einschließlich Applikatorkopf 38, Clipgreifer 27 und Antriebskopf 16 mit den Kulissenbahnen 17 gewechselt werden. Dabei ist es konstruktionsbedingt vorteilhaft, daß Applikator- und Antriebskopf gleichzeitig gewechselt werden und damit die Kulissenbahnen 17 stets den jeweiligen Clipabmessungen optimal angepaßt sind.

Wie bei den bekannten Ligaturclips üblich, besteht auch das hier vorgeschlagene System aus dem Clip und dem zugehörigen Applikator.

Ebenso wird in Anlehnung an die Ligaturclips für die bestifteten Clips ein mehrteiliger Clipsatz mit verschieden großen und für den jeweiligen Anwendungsfall optimal bemessenen Clips mit den entsprechend angepaßten Applikatoren oder Applikatorköpfen vorgeschlagen.

Der hier gewählte Bedienungsgriff gestattet den Antrieb von Applikatoren mit 5 mm, 7 mm und 10 mm Schaftaußendurchmesser in geeigneten Trokarhülsen. Der Wechsel der Instrumentenschäfte 21 erfolgt durch Bedienung der beiden Rändelschrauben 18 und 24 im Antriebskopf 16 bzw. Griffkopf 15.

Der Applikator besteht aus drei Baugruppen:
- Der Applikatorkopf 38 mit dem Maul 26, den Führungsnuten 42, 43 und 51, den Nachformungsrippen 40 und 41 und den Öffnerzapfen 47 dient in Verbindung mit dem Clipgreifer 27 zur Aufnahme und Formung des Clip.
- Der Handgriff besteht im wesentlichen aus dem Handtellergriffteil 14, dem über den Bolzen 25 gelenkig mit ihm verbundenen Fingergriffteil 13, den Flachfedern 52 für die Griffrückstellung, dem Griffkopf 15 mit dem schwenkbaren Federhebel 20 und der Flachfeder 50 zu seiner Rückstellung.
   Der Handgriff ist für Einhandbetrieb konzipiert. Er gestattet das Halten und Führen des Instruments, den Antrieb des Applikatorkopfes 38 und die Bedienung des Federhebeis 20 durch den Daumen des Operateurs bei der Abarbeitung des Applikationsprogrammes.
- Der Applikatorschaft 21 mit dem Schaftrohr 39, dem Übergangsstück 22, dem mit dem Übergangsstück verbundenen Antriebskopf 16 und dem Schaftinnenteil 23 ist in konstruktiv wählbarer Länge die Verbindung zwischen Applikatorkopf 38 bzw. Clipgreifer 27 und dem Handgriff. Der Antriebskopf 16 enthält zwei Kulissennuten 17, die im Zusammenspiel mit einer Zunge am Federhebel 20 ein sicheres Abarbeiten des Applikationsprogrammes ermöglichen. Die nebeneinander liegenden Kulissennuten 17 führen bei Bedienung des Applikatorgriffes die Zunge des Federhebels 20 auf drei verschiedenen Ebenen A, B und C, die in der Höhe um den Stufensprung s zueinander versetzt sind.

Im folgenden wird das Applikationsprogramm des nach Fig.1 vorgeschlagenen Clip im Detail dargestellt.

### Greifen und Laden des Clip

Der offene wie in Fig.3 gezeichnete Clip wird direkt aus einem geeignet geformten Magazin oder aus einer Pinzette vom Clipgreifer 27 übernommen. Bei proximaler Totpunktlage des Applikatorkopfes 38 ist, wie in Fig.6 dargestellt, der Clipgreifer 27 voll geöffnet. Der lichte Abstand zwischen den Greiferbacken 32 und den Greiferköpfen 29 ist in dieser Stellung maximal und durch die in diesem entspannten Zustand nach außen abgebogenen Flachfedern 31 vorgegeben.

Wenn der Brückenteil 8 am distalen Ende der Anschlagstange 28 anliegt, kann der Applikatorkopf 38 distalwärts verschoben werden. Entsprechend dem zurückgelegten Weg des Applikatorkopfes 38 relativ zu den Greiferköpfen 29 wird der Clipgreifer 27 geschlossen.

Bei der Greiferbeladung aus einem angepaßten Magazin ist sichergestellt, daß bei diesem Vorgang der Clip relativ zu den Greiferköpfen 29 exakt so positioniert ist, daß der Brückenteil 8 genau zwischen den proximalen Flächen der Greiferbacken 32 und dem distalen Ende der Anschlagstange 28 liegt. Ferner ist dann gewährleistet, daß die Lage des Clip in den Greiferköpfen 29 sowohl durch die Führung der Versatzflächen 7 des Clipunterteils 1 an den Indexflächen 34 der Greiferköpfe 29 als auch durch die Führung der Versatzflächen 6 des Clipoberteils 2 an den Indexflächen 33 fixiert ist.

Da diese exakte Zuordnung von Clip und Clipgreifer bei Freihandbeladung nicht automatisch gesichert ist, muß in solchen Fällen besondere Sorgfalt angewandt werden.

Nach Positionierung des Clip im offenen Greifer 27 können die Greiferbacken 32, wie beschrieben, durch distal gerichteten Vorschub des Applikatorkopfes 38 geschlossen werden.

Am Ende des Schließvorganges gleitet der Abschnitt des Clipoberteiles 2 zwischen den Zapfenrücken 5 und den Versatzflächen 6 sowie ein Teil des noch nicht geformten Scharnierbogens 48 des Brückenteils 8 durch den lichten Spalt zwischen den Öffnerzapfen 47 im Applikatorkopf 38 hindurch. Dabei ist konstruktiv ein hinreichender Abstand zwischen den Zapfenrücken 5 des noch gestreckten Clipoberteiles 2 und den Deckflächen 45 der Öffnerzapfen 47 gewährleistet.

Die Führungsrippen 30 an den Greiferköpfen 29 spuren ebenfalls in die Führungsnut 51 ein und sichern jetzt durch Abstützung der Greiferköpfe 29 an den Wänden der Nut 51 den Greifer 27 gegen Aufklaffen unter Belastung.

Die Führungsrippen 30 gleiten unter den Öffnerzapfen 47 im Applikatorkopf vorbei.

Die Greiferköpfe 29 mit dem Clipunterteil 1 werden dabei in die untere Führungsnut 42 des Applikatorkopfes 38 eingezogen.

Bis zur Berührung des Clipoberteiles 2 mit der Anschlag- und Formungsrippe 44 im Applikatorkopf 38 bleibt die Clipform, wie in Fig.3 dargestellt, unverändert.

Durch weiteres Vorschieben des Applikatorkopfes 38 nach distal relativ zum Clipgreifer 27 wird die biegeweiche Brücke 8 im Bereich des Scharnierbogens 48 zwischen der Anschlag- und Formungsrippe 44 und den Greiferbacken 32 so verformt, daß das Clipoberteil 2 eine Schließbewegung in Richtung Clipunterteil 1 beginnt. Der Clip hat dann etwa die in Fig.5 gezeichnete Form. Das Clipoberteil 2 liegt aber noch an der Anschlag- und Formungsrippe 44.

Während der bisher beschriebenen translatorischen Applikatorkopfbewegung ist die Zunge am Federhebel 20 zunächst auf einer ansteigenden Fläche der ersten Kulissenbahn 17a gegen die Federkraft des eigenen Schaftes von der Ausgangsebene A um 2 s auf das Niveau B gehoben worden. Fig.3 zeigt die entsprechende Clipposition im Applikatorkopf. Nach der oben beschriebenen ersten leichten Verformung des Brückenteiles 8 entsprechend der Fig.5 springt die Zunge am Federhebel 20 durch die Federwirkung des gespannten Hebelschaftes vom Niveau B um s auf die Ebene C der ersten Kulissenbahn 17 hinab.

Damit ist der Ladevorgang des Applikators beendet. Das Fingergriffteil 13 ist über die Blockade des Antriebskopfes 16 gegen Rückstellung in die Ausgangslage gesichert. Eine unbeabsichtigte Freigabe des Clip ist ausgeschlossen.

### Schließen und Öffnen des Clip

Wird der Applikatorkopf 38 aus der Ladeendstellung relativ zum Greifer 27 weiter nach distal verschoben, so wird die gegen Ende des Ladevorganges eingeleitete Schließbewegung des Clip fortgesetzt. Der Clip gleitet weiter in die Führungsnuten 42 und 51 ein. Die Brücke 8 am Clipoberteil 2 wird dabei durch das Kräftepaar zwischen den Greiferbacken 32 und der Anschlag- und Formungsrippe 44 zum Scharnierbogen 48 ausgebildet.

Die seitlich aus dem Clipoberteil 2 vorspringenden Zapfen 3 treten durch den Spalt zwischen den Öffnerzapfen 47 und der Anschlag- und Verformungsrippe 44 hindurch in die Führungsnut 51, an deren Seitenflächen sie entlang gleiten und dadurch das Clipoberteil 2 seitlich abstützen. Eine zusätzliche Seitenführung des Clipoberteiles 2 ist im Laufe des Schließvorganges durch sein Eintauchen zwischen die Öffnerzapfen 47 im Applikatorkopf 38 gegeben (Fig.4).

Die in Fig.4 gezeichnete Form des Clip, die auch bei weiterer Verschiebung des Applikatorkopfes bis zur Berührung mit den Anlaufflächen 49 der Nachformungsrippen 40 und 41 unverändert bleibt, erlaubt das Einbringen des Clip und des Applikatorkopfes 38 durch eine entsprechende Trokarhülse in den Operationssitus, da die Stirnsilhouette des Clip jetzt an keiner Stelle die Außenkontur des Applikatorkopfes überragt.

Der distale Vorschubweg des Applikatorkopfes 38 relativ zum Clipgreifer 27 wird durch den Anschlag der Federhebelzunge am proximalen Ende der ersten Kulissenbahn 17 auf der Ebene C im Antriebskopf 16 begrenzt. Ein unbeabsichtigtes Verriegeln des Clip bei Unterfahren der Nachformungsrippen 40 und 41 ist damit ausgeschlossen. Solange der Applikatorkopf 38 relativ zum Clipgreifer 27 in einem Bereich bewegt wird, der durch den distalen und proximalen Anschlag auf der Ebene C der ersten Kulissenbahn 17 begrenzt ist, bleibt der Clip gegen unbeabsichtigtes Freigeben und Verriegeln gesichert.

Zum Greifen und Aufladen des ersten Geweberandes muß der Clip im Operationsfeld geöffnet werden. Dazu wird das Fingergriffteil 13 entlastet und durch die entsprechend bemessenen Flachfedern 52 zwischen den Griffteilen zurückbewegt. Der Applikatorkopf 38 wird dadurch nach proximal verschoben.

Wenn sich die Flächen der Abschrägung 4 an den Zapfen 3 des Clipoberteiles 2 und die Flächen 46 an den Öffnerzapfen 47 berühren, entsteht durch Zerlegung der parallel zur Applikatorachse wirkenden Schubkraft auf die Zapfen 3 am Clipoberteil 2 eine Kraftkomponente, die den Clip, wie in Fig.5 dargestellt, durch teilweise Streckung des Scharnierbogens 48 öffnet.

Die Spitze 10 des Stiftes 9 am Clipunterteil 1 wird nahe dem Inzisionsrand auf die Außenfläche (die Fläche, die ohne Inzision zum Applikator zeigen würde) des Gewebes gesetzt und durch axiale Bewegung des Stiftes versucht, das Gewebe zu durchstechen. Unterstützt wird dieser Versuch durch vorsichtige Längsbewegung des Applikators nach proximal. Dabei entsteht an der zur Clipbrücke hin geneigten Stiftspitze 10 eine zusätzliche Kraftkomponente in Richtung der Stiftachse.

Ist ein Durchstich infolge zu großer Gewebewiderstände auf diese Weise nicht möglich, so wird durch Anziehen des Fingergriffteiles 13 der Applikatorkopf 38 relativ zum Clipgreifer 27 distalwärts bewegt und dadurch der Clip wie oben beschrieben geschlossen.

Dabei wird der zwischen der Stiftspitze 10 und dem Durchbruch 11 im Clipoberteil 2 liegende Gewebequerschnitt wegen des minimierten elastischen Ausweichens und wegen der Gegenhaltekraft des Oberteiles leichter durchstochen und der Geweberand auf die Spitze aufgeladen.

Der im lichten Querschnitt des Clip liegende Geweberand wandert beim Schließen des Clip in das Maul 26 des Applikatorkopfes 38.

Nach erneutem Öffnen des Clip wird der zweite Geweberand von der Inzisionsfläche oder der Geweberückseite her wie beschrieben durchstochen und aufgeladen. Muß zur Unterstützung des Durchstichs der Clip erneut geschlossen werden, so kann das Fingergriffteil 13 bis zum Anschlag der Federhebelzunge am proximalen Ende der ersten Kulissenbahn 17 auf der Ebene C angezogen werden.

In dieser Griff- und Applikatorkopfstellung liegt die Außenseite des Scharnierbogens 48 dicht vor den Anlaufflächen 49 der Nachformungsrippen 40 und 41.

Damit ist das Aufladen und Fixieren der Geweberänder relativ zueinander beendet. Nun muß der mit biegeweicher Brücke 8 konzipierte Clip verriegelt werden, da er aufgrund seiner Konstruktion ohne diese Sicherung nicht fähig ist, wie ein biegesteifer Ligaturclip die Gewebefixierungskräfte aufzunehmen.

### Verriegeln und Freigeben des beladenen Clip

Zur formschlüssigen Verriegelung des beladenen Clip wird bei geschlossenem Clip nach Fig.4 mit dem Daumen der griffhaltenden Hand der Federhebel 20 gegen den Widerstand der Flachfeder 50 zum Griffkopf 15 hin gedrückt.

Dadurch fällt die Zunge des Federhebels 20 um s auf das Niveau A in der zweiten Kulissenbahn 17 zurück. Nach diesem kurzen einmaligen Druck auf den Federhebel 20 ist die Zunge in der Kulissenbahn 17 gefangen. Sie gleitet am Stufensprung zwischen den Nuten 17 entlang und hindert dadurch den Federhebel 20 vor der Freigabe des Clip an der Rückstellung.

Wird das Fingergriffteil 13 jetzt weiter angezogen und damit der Applikatorkopf 38 relativ zum Clipgreifer 27 weiter nach distal verschoben, so berührt zunächst der Scharnierbogen 48 des Clip die schrägen Anlaufflächen 49 der Nachformungsrippen 40 bzw. 41.

Wenn die auftreibenden Gewebekräfte am Clipverschluß nicht zu groß sind, bewegt sich das distale Ende des Clipoberteiles 2, wie in Fig.7 dargestellt, unter Deformation des Scharnierbogens 48 weiter in Richtung Clipunterteil 1, bis sich der untere Rand des Durchbruches 11 auf die schrägen Kanten der Stiftspitze 10 aufsetzt.

Unter weiterem distalen Vorschub des Applikatorkopfes 38 wird der Scharnierbogen 48 unter die Nachformungsrippen 40 bzw. 41 gedrückt und dadurch das Clipoberteil 2 in die in Fig.8 dargestellte Form gebracht.

Dieser Vorgang hat eine distale Verschiebung des Durchbruchs 11 im Clipoberteil 2 relativ zum Brückenteil 8 zur Folge. Deshalb wandert auch die im Durchbruch 11 gefesselte Stiftspitze 10, wie in Fig.8 gezeichnet, distalwärts aus.

Während der Umformung des Scharnierbogens 48 wird auch die Stiftspitze 10 an der Anschlag- und Formungsrippe 44 umgebogen und gleitet in die Führungsnut 51.

Die Protektornase 12 am Clipoberteil 2 wird je nach Dicke und elastischer Gegenkraft des Gewebes im Clip mehr oder weniger gerade gebogen. Die Rückstellkräfte der Protektornase 12 nach der Verformung sichern durch das Abstützen des Lochrandes im Clipoberteil 2 auf den Kanten der Stiftspitze 10 das Aufliegen der Spitze auf der Protektornase und damit das Lagergewebe des Clip gegen Verletzung.

Die bisher beschriebenen Verformungen werden unter Verschiebung des Applikatorkopfes 38 und des Schaftrohres 39 bis zum Anschlag der Zunge des Federhebels 20 am proximalen Ende der Kulissennut 17 erzeugt.

Das Clipoberteil 2 ist durch den Umformungs- und Schließvorgang aus dem Grund der Führungsnut 51, in dem durch translatorisches Gleiten das Öffnen und Schließen des Clip realisiert wurde, weiter zur Achse des Applikatormaules 26 versetzt worden (Fig.8). Die Zapfen 3 am Clipoberteil 2 liegen jetzt, wie in Fig.8 dargestellt, in einer Ebene unterhalb der Öffnerzapfen 47. Wird der Applikatorkopf 38 nach proximal zurückgezogen, so gleiten die Zapfen 3 am Clipoberteil 2 unter den Öffnerzapfen 47 hindurch.

Nach weiterem Zurückziehen des Applikatorkopfes 38 öffnen die Flachfedern 31 die die Cllpbrücke 8 umfassenden Greiferköpfe 29, und die Greiferbacken 32 geben den entsprechend Fig.1 geformten Clip mit den eingeschlossenen Geweberändern frei.

Gegen Ende des Freigabevorganges springt durch den Druck der Flachfelder 50 die Zunge des Federhebeis 20 auf dem Niveau A von der zweiten Kulissenbahn 17 in die erste Kulissenbahn 17 zurück. Damit befindet sich der Federhebel 20 wieder in der Ausgangsstellung.

Der Applikator kann mit vorstehendem Greifer 27 durch die Trokarhülse aus dem Operationssitus entfernt und sofort mit dem nächsten Clip beladen werden.

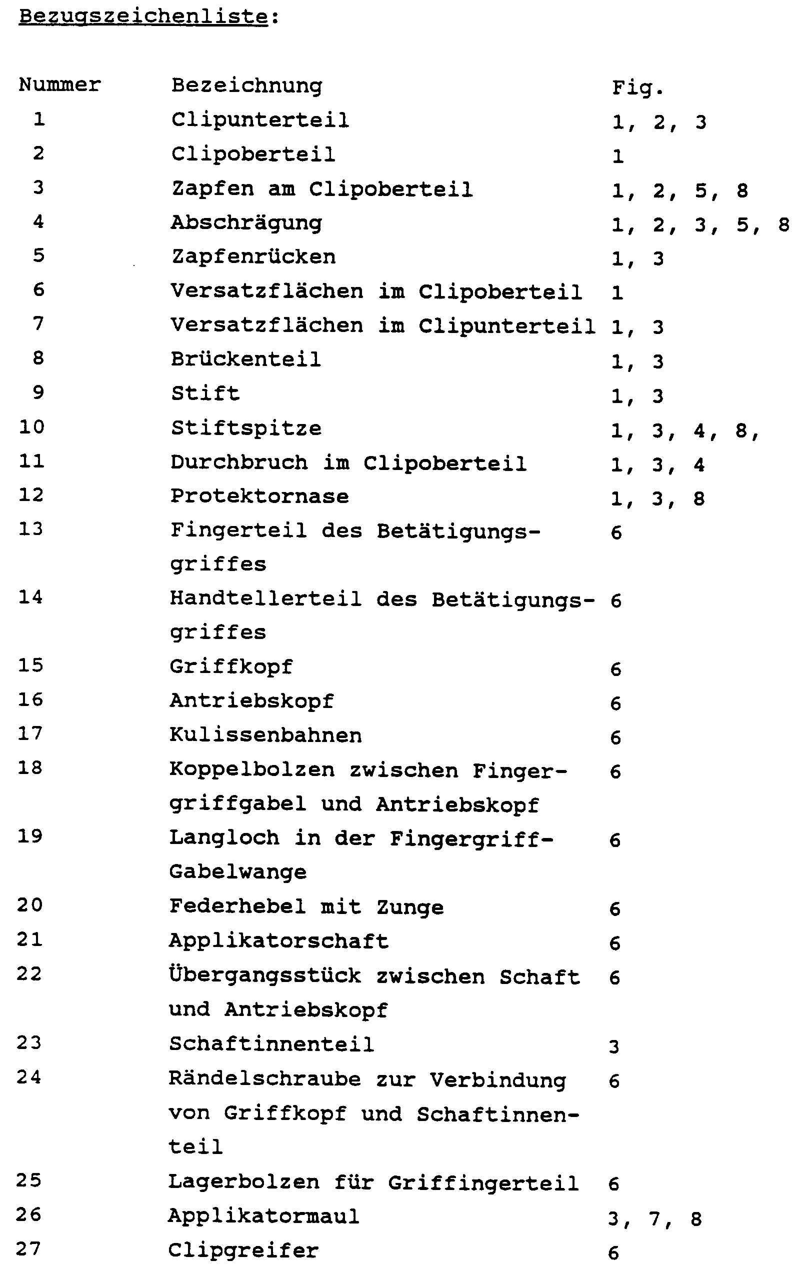

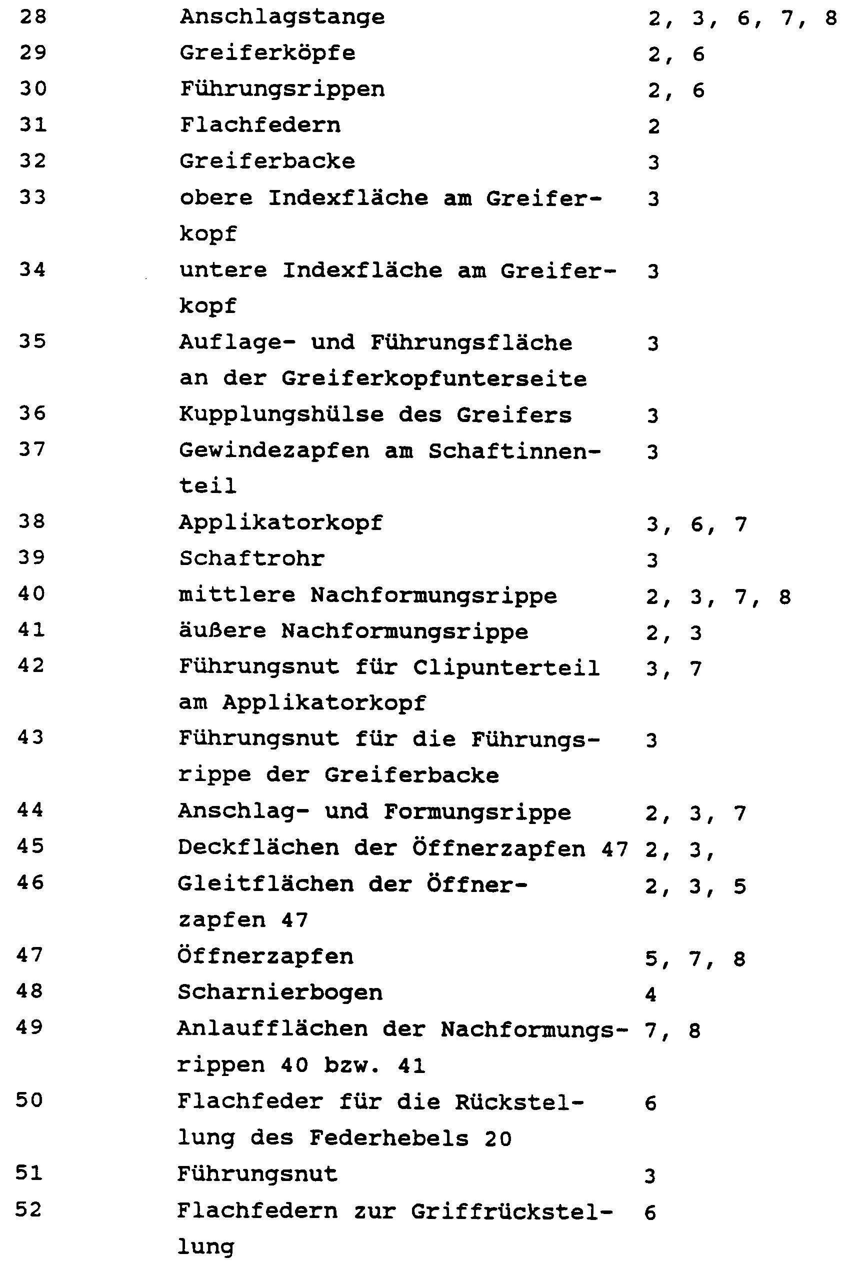

## Patentansprüche

1. Clip zur Anwendung in der Chirurgie mit den Merkmalen:
a) der Clip besteht aus einem Clipunterteil (1) und einem Clipoberteil (2), die
b) durch ein biegeweiches Brückenteil (8) miteinander verbunden sind;
c) das Clipunterteil (1), das Clipoberteil (2) und das biegeweiche Brückenteil (8) liegen in einer Symmetrieebene;
d) das Clipunterteil (1) trägt an seinem freien Ende einen in der Symmetrieebene abgewinkelten, mit einer Spitze (10) versehenen Stift (9);
e) das Clipoberteil (2) trägt an seinem freien Ende eine verrundete Protektornase (12),
f) an die sich ein in der Symmetrieebene angeordneter Durchbruch (11) anschließt;
g) die Längen des Clipunterteils (1) einschließlich des Stiftes (9), des Clipoberteils (2) bis zum Durchbruch (11) und des biegeweichen Brückenteils (8) sind so gewählt, daß die Spitze (10) des Stiftes (9) durch Biegen des Clipoberteils (2) unter Verformung des biegeweichen Brückenteils (8) in Richtung auf das Clipunterteil (1) in den Durchbruch (11) einführbar ist,
gekennzeichnet durch die Merkmale:
h) der Stift (9) verjüngt sich zur Spitze (10), wobei die Spitze eine kegel- oder pyramidenförmige Gestalt aufweist;
i) die Länge des biegeweichen Brückenteils (8) ist so bemessen, daß es zum Schließen des Clips in der Symmetrieebene an zwei verschiedenen Stellen zu jeweils einem annähernd rechten Winkel in der Weise verformbar ist, so daß der geschlossene Clip annähernd die Form eines Rechtecks annimmt, dessen Seiten das biegeweiche Brückenteil (8), das Clipunterteil (1), der Stift (9) und das Clipoberteil (2) darstellen.

2. Clip nach Anspruch 1, dadurch gekennzeichnet, daß die Protektornase (12) in die selbe Richtung wie der Stift (9) abgewinkelt ist.

3. Clip nach Anspruch 1 oder 2, dadurch gekennzeichnet, daß der Querschnitt des biegeweichen Brückenteils (8) gegenüber den Querschnitten des Clipunterteils (1) und des Clipoberteils (2) vermindert ist.

4. Clip nach Anspruch 3, dadurch gekennzeichnet, daß die Grenzflächen zwischen dem Clipunterteil (1), dem Clipoberteil (2) und dem biegeweichen Brückenteil (8) als Versatzflächen (6,7) ausgebildet sind.

5. Clip nach einem der Ansprüche 1 bis 4, dadurch gekennzeichnet, daß das Clipoberteil (2) in seinem an das biegeweiche Brückenteil (8) angrenzenden Bereich zwei symmetrisch senkrecht zur Symmetrieebene angeordnete Zapfen (3) trägt.

6. Clipapplikator mit einem Clip gemäß einem der Ansprüche 1 bis 5, gekennzeichnet durch
a) einen Clipgreifer (27) zum Ergreifen und Festhalten des biegeweichen Brückenteils (8) des Clip;
b) ein Applikator-Schaftrohr (39), das über einen Betätigungsgriff (13, 14) axial und senkrecht zum festgehaltenen biegeweichen Brückenteil (8) des Clip verschiebbar ist und dessen Kopf (38) den Clip bei distalem Vorschub im Bereich des biegeweichen Brückenteils (8) in der Weise verformt, daß das Clipunterteil (1) und das Clipoberteil (2) eine angenähert parallele Stellung einnehmen, wobei die Spitze (10) des Stiftes (9) den Durchbruch (11) durchstößt;
c) zwei Öffnerzapfen (47) im Applikatorkopf (38) und eine Anschlagstange (28) im Clipgreifer (27), mit deren Hilfe bei proximaler Bewegung des Applikator-Schaftrohres (39) das Clipoberteil (2) durch Verformen des biegeweichen Brückenteils (8) in eine annähernd senkrechte Stellung zum Clipunterteil (1) gebracht werden kann.

## Claims

1. Clip for surgical use, having the following features:
a) the clip comprises a lower clip portion (1) and an upper clip portion (2), which portions
b) are interconnected by a flexible bridge member (8);
c) the lower clip portion (1), the upper clip portion (2) and the flexible bridge member (8) lie in a plane of symmetry;
d) the lower clip portion (1) carries, at its free end, a pin (9) which is bent-over in the plane of symmetry and is provided with a point (10);
e) the upper clip portion (2) carries, at its free end, a rounded protective projection member (12),
f) which communicates with an opening (11) disposed in the plane of symmetry;
g) the length of the lower clip portion (1), including the length of the pin (9), the length of the upper clip portion (2) as far as the opening (11), and the length of the flexible bridge member (8) are selected so that the point (10) of the pin (9) is insertable into the opening (11) as a result of bending the upper clip portion (2) whilst deforming the flexible bridge member (8) in a direction towards the lower clip portion (1),
characterised by the following features:
h) the pin (9) tapers towards the point (10), the point having a conical or pyramid-shaped configuration;
i) the length of the flexible bridge member (8) is dimensioned so that, for the closure of the clip, it is deformable in the plane of symmetry at two different locations at an approximate right angle in such a manner that the closed clip approximately assumes the shape of a rectangle, the sides of which are represented by the flexible bridge member (8), the lower clip portion (1), the pin (9) and the upper clip portion (2).

2. Clip according to claim 1, characterised in that the protective projection member (12) is bent-over into the same direction as the pin (9).

3. Clip according to claim 1 or 2, characterised in that the cross-section of the felexible bridge member (8) is smaller than the cross-sections of the lower clip portion (1) and the upper clip portion (2).

4. Clip according to claim 3, characterised in that the interfaces between the lower clip portion (1), the upper clip portion (2) and the flexible bridge member (8) are configured as offset faces (6, 7).

5. Clip according to one of claims 1 to 4, characterised in that the upper clip portion (2) carries, in its region abutting against the flexible bridge member (8), two pins (3) which are symmetrically disposed perpendicular to the plane of symmetry.

6. Clip applicator having a clip according to one of claims 1 to 5, characterised by
a) a clip gripping device (27) for gripping and retaining the flexible bridge member (8) of the clip;
b) an applicator shank tube (39), which is displaceable, via an operating handle (13, 14), axially and perpendicularly to the retained, flexible bridge member (8) of the clip, and the head (38) of which tube deforms the clip in the event of distal advancement in the region of the flexible bridge member (8) in such a manner that the lower clip portion (1) and the upper clip portion (2) assume an approximately parallel position, the point (10) of the pin (9) piercing the opening (11);
c) two opening pins (47) in the applicator head (38) and one stop rod (28) in the clip gripping device (27), by means of which component parts the upper clip portion (2) can be brought into an approximately perpendicular position relative to the lower clip portion (1) by deforming the flexible bridge member (8) in the event of a proximal movement of the applicator shank tube (39).

## Revendications

1. Pince pour utilisation en chirurgie ayant les caractéristiques :
a) la pince se compose d'une partie inférieure (1) et d'une partie supérieure (2), qui
b) sont reliées ensemble par une partie de pont souple et pliable (8),
c) la partie inférieure (1), la partie supérieure (2) et la partie de pont (8) souple et pliable sont situées dans un plan de symétrie,
d) la partie inférieure (1) porte à son extrémité libre une tige (g) se développant dans le plan de symétrie, munie d'une pointe (10),
e) la partie supérieure (2) porte à sont extrémité libre un nez protecteur arrondi (12),
f) auquel se raccorde un passage (11) disposé dans le plan de symétrie,
g) les longueurs de la partie inférieure de pince (1) y compris la tige (9), la partie supérieure de pince (2) jusqu'au passage (11) et la partie de pont souple et pliable (8) sont choisies, de sorte que la pointe (10) de la tige (9) durant le cintrage de la partie supérieure (2) de la pince soit insérable dans le passage (11) par déformation de la partie de pont (8) souple et pliable en direction de la partie inférieure de pince (1),
caractérisé par les caractéristiques :
h) la tige (9) s'amincit à la pointe (10), la pointe comportant une structure en forme de cône ou de pyramide,
i) la longueur de la partie de pont souple et pliable (8) est dimensionnée pour qu'elle soit déformable à la fermeture de la pince dans le plan de symétrie en deux endroits différents en un angle approximativement droit de manière, que la pince fermée prenne approximativement la forme d'un rectangle, dont les côtés représentent la partie de pont (8) souple et pliable, la partie inférieure de pince (1), la tige (9) et la partie supérieure de pince (2).

2. Pince selon la revendication 1,
caractérisée en ce que
le nez protecteur (12) est coudé dans le même sens que la tige (9).

3. Pince selon les revendications 1 ou 2,
caractérisée en ce que
la section transversale de la partie de pont (8) souple et pliable est réduite par rapport aux sections transversales de la partie inférieure (1) et de la partie supérieure (2) de la pince.

4. Pince selon la revendication 3,
caractérisée en ce que
les surfaces limites entre la partie inférieure (1), la partie supérieure (2) de la pince et la partie de pont souple et pliable (8) sont réalisées comme des surfaces de déplacement (6, 7).

5. Pince selon une des revendications 1 à 4,
caractérisée en ce que
la partie supérieure de pince (2) porte dans sa zone avoisinant la partie de pont (8) souple et pliable deux goujons (3) disposés symétriquement perpendiculaires au plan de symétrie.

6. Applicateur de pince avec une pince selon une des revendications 1 à 5,
caractérisé par
a) un organe de prise de pince (27) pour saisir et maintenir la partie de pont (8) souple et pliable de la pince,
b) un tube tige applicateur (39), qui est déplaçable axialement et perpendiculairement à la partie de pont (8) souple et pliable et maintenu fixe de la pince et dont la tête (38) déforme la pince lors de la poussée distale dans la zone de la partie de pont (8) souple et pliable de manière, que la partie inférieure (1) et la partie supérieure (2) de la pince prennent une position approximativement parallèle, la pointe (10) de la tige (9) passant à travers le passage (11),
c) deux goujons d'ouverture (47) dans la tête applicateur (38) et une barre de butée (28) dans l'organe de prise de la pince (27), à l'aide desquels dans le mouvement proximal du tube tige de l'applicateur (39) la partie supérieure de la pince (2) peut être amenée dans une position approximativement perpendiculaire à la partie inférieure de la pince (1) par la déformation de la partie de pont souple et pliable (8).
